# EUROPÄISCHE PATENTSCHRIFT

(11) **EP 4 309 634 B1**
(45) Veröffentlichungstag und Bekanntmachung des Hinweises auf die Patenterteilung: **14.05.2025**
(21) Anmeldenummer: 23186380.4
(22) Anmeldetag: 19.07.2023
(51) Int. Cl.: A61F 7/02

(54) **TEMPERIERVORRICHTUNG ZUR DERMALEN APPLIKATION**
TEMPERING DEVICE FOR DERMAL APPLICATION
DISPOSITIF DE RÉGULATION DE TEMPÉRATURE POUR APPLICATION DERMIQUE

(30) Priorität: 19.07.2022 DE 202022104070 U
(43) Veröffentlichungstag der Anmeldung: 24.01.2024
(73) Patentinhaber: AUROX GmbH, 8010 Graz (AT)
(72) Erfinder: Schöggler, Christoph, 8010 Graz (AT)
(74) Vertreter: Isarpatent

(56) Entgegenhaltungen:
- CN-A- 107 845 723
- JP-A- H06 197 924
- KR-A- 20100 028 911
- US-A1- 2006 168 969
- US-A1- 2018 172 325

## Beschreibung

Die Erfindung betrifft Temperiervorrichtungen, welche temporär dermal am menschlichen Körper applizierbar sind.

Thermoelektrische Kühlung wird in zahlreichen Bereichen der Wissenschaft und Technik eingesetzt, insbesondere in der Medizin. Thermoelektrische Kühlung kann in praktischen Anwendungen, wie etwa in der Kryochirurgie, Kryotherapie, Neurochirurgie, Urologie, plastischen Chirurgie und Dermatologie genutzt werden. In der Dermatologie können thermoelektrische Geräte eingesetzt werden, um Falten zu glätten, Teile des menschlichen Körpers zu stärken oder für ein verbessertes Wohlbefinden zu sorgen.

Im periokularen Gesichtsbereich kutan applizierbare und wieder entfernbare Auflageelemente, auch "Augenpads" genannt, sind Produkte aus dem Bereich der Kosmetikindustrie, welche von einem Benutzer oder einer Benutzerin im Bereich unter den und/oder um die Augen angebracht werden, um einen kosmetischen Effekt an der Haut des Benutzers zu bewirken. Auch im kutanen Bereich zwischen Nase, Nasolabialfalten und Oberlippe, dem Philtrum, können kutan applizierbare Auflageelemente, auch "Philtrumpads" genannt, zur Verminderung der Faltenbildung eingesetzt werden. Insbesondere gegen Plissee-Fältchen kann die gezielte kutane Applikation von Wärme bzw. Kälte ein probates Mittel darstellen.

In ähnlicher Weise können aktive Temperiervorrichtungen zur Kühlung einer Körperregion, wie etwa eines Kopfbereiches oder eines Bereiches der äußeren Gliedmaßen eingesetzt werden, welche ein wärmeleitfähiges Hautkontaktmaterial und ein das Hautkontaktmaterial einstellbar temperierendes Peltierelement aufweisen.

Im Stand der Technik sind Vorrichtungen bekannt, welche die Haut unter den Augen eines Benutzers erwärmen und/oder kühlen. Herkömmlicherweise werden hierfür natürliche Mittel wie beispielsweise Gurkenscheiben oder Ähnliches verwendet, welche eine Kühlwirkung durch abgeführte Verdunstungswärme bewirken. Es sind jedoch auch technische Vorrichtungen bekannt, welche auf die Haut aufgebracht werden und mittels elektrischer, chemischer oder biochemischer Komponenten eine Erwärmung oder Kühlung der Haut bewirken.

Beispiele hierfür werden in den Druckschriften US 301,931 A, US 4,585,002 A, US 8,525,363 B1, US 2013/0172829 A1, US 9,849,024 B2, US 10,973,275 B2, US 2021/0127769 A1 oder WO 2018/122710 A1 beschrieben.

Die Druckschrift US 2006/0168969 A1 offenbart eine thermoelektrische Vorrichtung mit einem thermoelektrischen Peltier-Modul, einer Wärmesenke auf der heißen Seite, einer Wärmesenke auf der kalten Seite und einem Temperatursensor wie einem Thermistor mit negativem Temperaturkoeffizienten (NTC), der direkt in das thermoelektrische Modul eingebaut ist.

Die Druckschrift KR 2010-0028911 A offenbart eine Kühlvorrichtung, die ein thermoelektrisches Modul, einen ersten Thermistor, der an der Kühleinheit des thermoelektrischen Moduls angebracht ist, einen zweiten Thermistor, der an dem wärmeerzeugenden Teil des thermoelektrischen Moduls angebracht ist, und eine Steuerung, die so konfiguriert ist, dass sie einen Treiberstrom des thermoelektrischen Moduls auf der Grundlage von Temperaturen steuert, die von dem ersten und dem zweiten Thermistor gemessen werden, aufweist.

Die Druckschrift US 2018/0172325 A1 offenbart ein thermoelektrisches System, das zwei voneinander beabstandete Substrate, elektrisch verbundene Halbleiterelemente in einem Spalt zwischen den zwei Substraten, einen Sensor zwischen den Substraten, und eine Dichtung, die sich zwischen den Substraten erstreckt und den Sensor und mindestens eines der mehreren Halbleiterelemente umschließt, aufweist.

Die Druckschrift JP H06-197924 A offenbart ein thermotherapeutisches Instrument, welches auf seiner Oberflächenseite einen vertieften Teil bildet, und ein Heizelement aufweist, das in dem therapeutischen Instrument Wärme abgibt und die Unterseite des vertieften Teils erwärmt.

Die Druckschrift CN 107 845 723 A offenbart eine thermoelektrisch arbeitende Vorrichtung.

Eine Aufgabe der vorliegenden Erfindung ist es, eine zur dermalen Applikation vorgesehene aktive Temperiervorrichtung für ein definiertes Erwärmen beziehungsweise Kühlen von Körperregionen bereitzustellen.

Gemäß einem Aspekt der Erfindung umfasst eine Temperiervorrichtung zur dermalen Applikation eine insbesondere elektrisch nicht leitfähige Hautkontaktfläche und zumindest ein mit der Hautkontaktfläche verbundenes Peltierelement. Die Hautkontaktfläche kann beispielsweise durch eine elektrisch nicht leitfähige dünne Folie oder eine elektrisch nicht leitfähige dünne Beschichtung aus einem geeigneten elektrisch nicht leitfähigen Material gebildet werden, welche auf einer Seite des Peltierelements aufgebracht ist. Das Peltierelement weist zwei parallele, voneinander beabstandete Isolationsplatten auf, zwischen denen dotierte Halbleiterelemente eingebracht sind, durch die elektrischer Strom geleitet werden kann. Ferner weist die Temperiervorrichtung einen Temperatursensor auf, welcher in einer Aussparung des Peltierelements zwischen den Isolationsplatten integriert und mit einer Temperaturmessschaltung außerhalb des Peltierelements verbunden ist.

Erfindungsgemäß weist der Temperatursensor eine Leiterplatte und einen auf einer Seite der Leiterplatte angebrachten negativen Temperaturkoeffizient-(NTC)-Sensor auf. Erfindungsgemäß weist der Temperatursensor ferner eine Spangenfedereinrichtung auf, welche auf einer von dem NTC-Sensor abgewandten Seite der Leiterplatte befestigt ist.

Gemäß einiger Ausführungsvarianten der Temperiervorrichtung ist die elektrisch nicht leitfähige Hautkontaktfläche eine Beschichtung oder Folierung der nach außen gekehrten Isolationsplatte des Peltierelements. In einigen Ausführungsvarianten dient die Beschichtung oder Folierung zum mechanischen Schutz der Oberfläche der Isolationsplatte des Peltierelements. Die Beschichtung im Sinne dieser Anmeldung kann beispielsweise durch das Aufbringen einer festhaftenden Schicht aus formlosem Stoff auf die Oberfläche der Isolationsplatte des Peltierelements gebildet werden. Bei der Beschichtung kann es sich sowohl um einzelne Schichten als auch um mehrere in sich zusammenhängende Schichten handeln. Dabei kann die Beschichtung in einem chemischen, mechanischen, thermischen und/oder thermomechanischen Beschichtungsverfahren aufgetragen sein. Auf diese Weise können mechanische Beschädigungen wie Kratzer oder Farbabtrag an dem Peltierelement vermieden werden und/oder der Korrosionsschutz erhöht werden.

Gemäß einer Weiterbildung der Temperiervorrichtung ist die Beschichtung oder Folierung vollständig plan ausgebildet. Gemäß einer Weiterbildung der Temperiervorrichtung weist die Beschichtung ein keramisches Material auf. Insbesondere ist die Beschichtung als Keramiklack ausgebildet, beispielsweise Cerakote^{®} von NIC Industries, Oregon, USA. Alternativ oder zusätzlich weist die Beschichtung beispielsweise ein Substrat auf, welches vor dem Beschichtungsvorgang als Flüssigkeit, insbesondere als Lack, oder als Feststoff, insbesondere als Pulver, Laminatfolie oder vergleichbare Folie, ausgebildet ist.

Gemäß einiger Ausführungsvarianten der Temperiervorrichtung weist die Temperiervorrichtung ferner eine Fixiereinrichtung auf, welche das Peltierelement an einer Gehäusebasis fixiert. Die Fixiereinrichtung ist beispielsweise aus einem glasfaserverstärkten Kunststoff hergestellt. Sie kann das Peltierelement abschnittsweise umgeben und mit der Gehäusebasis der Temperiervorrichtung befestigt sein. Dabei kann die Fixiereinrichtung mit der Gehäusebasis durch ein lösbares Befestigungsmittel, insbesondere durch eine Schraubverbindung, eine Steckverbindung oder dergleichen befestigt sein. Auf diese Weise kann das Peltierelement unter einem vorbestimmten Anpressdruck an die Gehäusebasis gepresst werden, so dass ein definierter Kontakt entsteht. Dabei kann die Gehäusebasis alle Komponenten der Temperiervorrichtung tragen. Die Gehäusebasis kann alternativ oder zusätzlich dazu aus einem temperaturleitenden Material gefertigt werden. Hierdurch wird die Wärmeabfuhr von der von der Hautkontaktfläche abgewandten Seite des Peltierelements an die Umgebung verbessert, wenn die Hautkontaktfläche mit der kalten Seite des Peltierelements korrespondiert, so dass die nicht benötigte Wärme der warmen Seite nicht in der Temperiervorrichtung aufgestaut ist. Eine thermische Kopplung zwischen der Gehäusebasis und der von der Hautkontaktfläche abgewandten Seite des Peltierelements kann beispielsweise durch flächigen Kontakt vorgesehen sein. Zusätzlich kann eine Wärmeleitpaste, ein Wärmeleitpad oder ein vergleichbares wärmeleitendes Material etwaige Hohlräume in dem flächigen Kontakt ausfüllen und somit die Wärmeübertragung zwischen dem Peltierelement und der Gehäusebasis unterstützen.

Gemäß einer Weiterbildung der Temperiervorrichtung ist die Fixiereinrichtung derart kompakt ausgebildet, dass sie in einem montierten Zustand einen geringeren Abstand in Bezug zu der Gehäusebasis aufweist als die Hautkontaktfläche von der Gehäusebasis beabstandet ist.

Gemäß einer Weiterbildung der Temperiervorrichtung umschließt die Fixiereinrichtung das Peltierelement zwischen den Isolationsplatten formschlüssig zumindest abschnittsweise.

Gemäß einiger Ausführungsvarianten der Temperiervorrichtung ist eine Steuereinheit mit dem Temperatursensor und dem Peltierelement verbunden. Die Steuereinheit ist dazu ausgebildet, eine Heiz- beziehungsweise Kühlleistung des Peltierelements anhand einer von dem Temperatursensor erfassten Temperatur der Peltierelementoberfläche in dem Aufnahmebereich zu regeln. Ferner kann die Steuereinheit dazu ausgebildet sein, bei Erreichen einer Zieltemperatur der Peltierelementoberfläche in dem Aufnahmebereich die Heiz- beziehungsweise Kühlleistung des Peltierelements derart zu regeln, dass die Temperatur der Peltierelementoberfläche in dem Aufnahmebereich im Wesentlichen konstant bleibt. Dabei kann das Peltierelement wechselnd mit oder ohne elektrischer Spannung beaufschlagt sein, sodass das Peltierelement dabei zeitweise mit Strom durchflossen ist. Die Steuereinheit schaltet beispielsweise einen Stromkreis, in dem das Peltierelement und eine Stromquelle integriert sind, ein beziehungsweise aus in Abhängigkeit von der erfassten Temperatur im Vergleich zu der Zieltemperatur. Optional ist die Steuereinheit ferner dazu ausgebildet, eine von einem Umgebungstemperatursensor gemessene Umgebungstemperatur bei der geregelten Heiz- beziehungsweise Kühlleistung derart zu berücksichtigen, dass in vorbestimmten Temperaturbereichen der Umgebungstemperatur die Heiz- beziehungsweise Kühlleistung erhöht, verringert oder unterbunden wird. So kann exemplarisch in einer Umgebung, deren Temperatur höher ist als die Zieltemperatur, das weitere Erwärmen der Hautkontaktfläche verhindert werden. Optional kann in einer Umgebung mit Umgebungstemperatur unterhalb einer üblichen Raumtemperatur die Heizleistung erhöht werden, um die Verlustwärme über die Hautkontaktfläche an die Umgebung zu kompensieren.

Bevorzugte und alternative Ausführungsvarianten der erfindungsgemäßen Temperiervorrichtung werden in weiterer Folge anhand der Figuren näher erläutert.
Figur 1 zeigt eine im Schnitt dargestellte Seitenansicht einer Temperiervorrichtung mit einer vollständig plan ausgebildeten Beschichtung gemäß einem Ausführungsbeispiel der Erfindung;
Figur 2 zeigt eine schematische Darstellung von zwei Peltierelementen mit jeweils einem integrierten Temperatursensor gemäß einem Ausführungsbeispiel der Erfindung;
Figur 3 zeigt eine schematische Seitenansicht eines Temperatursensors gemäß einem Ausführungsbeispiel der Erfindung in einem unverbauten Zustand;
Figur 4 zeigt eine im Schnitt dargestellte Seitenansicht eines Peltierelements mit einem zwischen den zwei Isolationsplatten des Peltierelements integrierten Temperatursensor gemäß einem Ausführungsbeispiel der Erfindung;
Figur 5 zeigt eine schematische Perspektivansicht eines Peltierelements mit einer Fixiereinrichtung, welche das Peltierelement zwischen den zwei Isolationsplatten formschlüssig zumindest abschnittsweise umschließt, gemäß einem Ausführungsbeispiel der Erfindung.

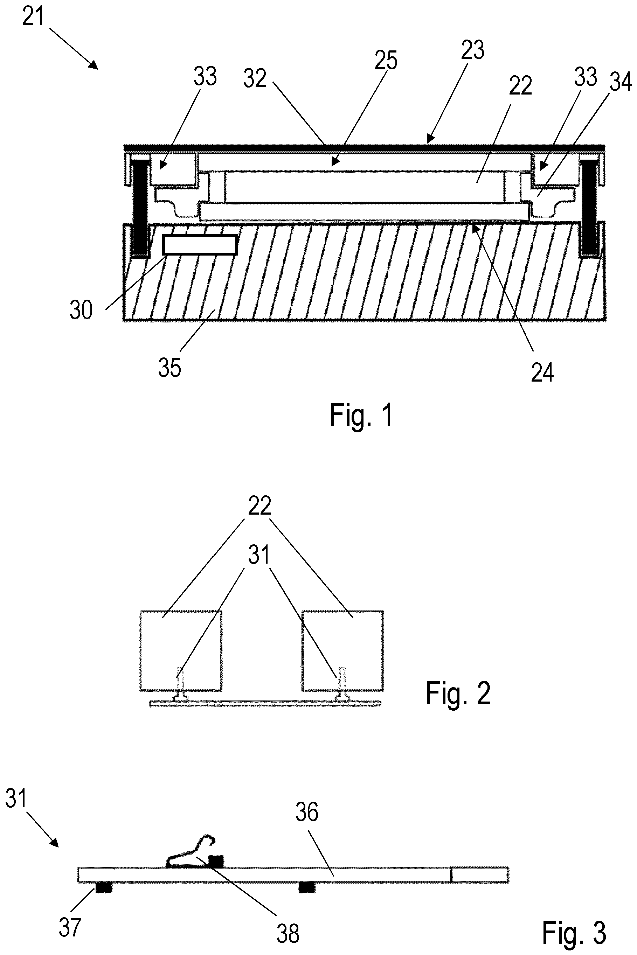

In Figur 1 zeigt eine im Schnitt dargestellte Seitenansicht einer Temperiervorrichtung 21 mit einer vollständig plan ausgebildeten Beschichtung. Die Temperiervorrichtung 21 ist für eine temporäre dermale Applikation des menschlichen Körpers vorgesehen, beispielsweise im Gesichtsbereich oder im Bereich der äußeren Gliedmaßen wie etwa Oberarme, Schultern, Waden oder dergleichen, so dass durch die Temperiervorrichtung 21 erzeugte Wärme bzw. Kälte auf die Haut im Applikationsbereich übertragen werden kann.

Die Temperiervorrichtung 21 weist zumindest ein Peltierelement 22 auf. Ein Peltierelement 22 kann aus einer Vielzahl von zum Beispiel quaderförmigen oder zylindrischen Halbleiterelementen unterschiedlichen Dotierungstyps, beispielsweise Bismuttellurid oder Siliciumgermanium, gebildet werden, die sequentiell in alternierender Abfolge an der Ober- bzw. Unterseite durch elektrisch leitfähige Zwischenverbinder gekoppelt sind. Die elektrisch leitfähigen Zwischenverbinder bilden thermische Kontaktflächen und sind zwischen zwei Isolationsplatten, wie beispielsweise dünne Keramikplatten eingebettet. Bei Durchfluss eines vordefinierten elektrischen Stromes durch die Serienschaltung der alternierenden Halbleiterelemente kühlen sich die an der einen Isolationsplatte gelegenen Zwischenverbinder je nach Stromstärke und Stromrichtung ab, während sich die gegenüberliegenden Halbleiterelemente entsprechend aufwärmen. Dadurch wird eine vom Stromfluss getriebene Temperaturdifferenz zwischen den Isolationsplatten erzeugt.

Ferner weist das Peltierelement 22 beispielhaft eine Beschichtung oder Folierung 32 zum mechanischen und/oder chemischen Schutz der Peltierelementoberfläche auf. Die Beschichtung oder Folierung 32 ist exemplarisch als Laminatfolie oder Keramiklackierung ausgebildet. Dabei ist die Beschichtung oder Folierung 32 auf der in Fig. 1 exemplarisch nach oben weisenden Isolationsplatte 25 des Peltierelements 22 aufgebracht. Dabei ist das Peltierelement 22 in einem Aufnahmebereich 23 einer Gehäusebasis 35 eingebracht. Der Aufnahmebereich 23 entspricht einem zurückgesetzten Bereich der Gehäusebasis 35 aufgenommen, dessen Seitenwände an der Oberkante im Wesentlichen bündig mit der oberen Isolationsplatte 25 des Peltierelements 22 abschließen. Die Beschichtung oder Folierung 32 überspannt die Isolationsplatte 25 des Peltierelements 22 und erstreckt sich über die Isolationsplatte 25 des Peltierelements 22 hinaus auf die Oberkanten der Seitenwände der Gehäusebasis 35 erstrecken, so dass ein umlaufender Spalt zwischen der bündig mit der Gehäusebasis 35 abschließenden oberen Isolationsplatte 25 des Peltierelements 22 und den Seitenwänden der Gehäusebasis 35 fluiddicht abgeschlossen ist. Dadurch wird eine elektrisch nicht leitfähige Hautkontaktfläche 23 gebildet, deren Fläche größer als die Oberfläche der oberen Isolationsplatte 25 des Peltierelements 22 ist. Durch die fluiddichte Versiegelung der Spalten an der Hautkontaktfläche 23 wird die Auflagefläche auf der Haut gleichmäßiger und enger. Außerdem kann Fremdmaterial wie beispielsweise Schweiß oder Hauttalg nicht in den Spalt zwischen Gehäusebasis 35 und Peltierelement 22 eindringen, so dass eine Reinigung der Temperiervorrichtung 21 erleichtert, Fehlfunktionen minimiert und eine Heiz- bzw. Kühleffizienz der Temperiervorrichtung 21 verbessert werden können.

Im Rückgriff auf Fig. 1, kann die Temperiervorrichtung 21 optional einen aufladbaren elektrischen Energiespeicher aufweisen. Der Energiespeicher ist zumindest mit dem Peltierelement 22 elektrisch verbunden, kann zusätzlich aber auch mit weiteren Komponenten der Temperiervorrichtung 21 elektrisch verbunden sein. Der Energiespeicher kann beispielsweise eine Batterie oder ein Akkumulator sein, welcher kabelgebunden oder kabellos, insbesondere induktiv, resonant oder dergleichen, aufladbar sein kann. Weiter kann der Energiespeicher in der Temperiervorrichtung 21 fest oder auswechselbar integriert sein.

Die Gehäusebasis 35 kann das Peltierelement 22 und gegebenenfalls den elektrischen Energiespeicher aufnehmen bzw. haltern. Zudem kann die Gehäusebasis 35 ein temperaturleitendes Material aufweisen, um die Abwärme des Peltierelements 22 aufzunehmen und an die Umgebung abgeben zu können. Auf diese Weise kann eine Überhitzung der Temperiervorrichtung 21 verringert werden. Gleichermaßen können auch separate Kühlkörper in den Boden der Gehäusebasis 35 unter der unteren Isolationsplatte 24 einzulegen. Darüber hinaus kann die Gehäusebasis 35 einen Temperatursensor aufweisen, welcher die Temperatur der Basis misst. Somit kann die Temperatur der Basis als Bezugsgröße für die Temperatur der Peltierelementoberfläche herangezogen werden, um beispielsweise bei einer zu hohen Temperatur der Basis das Peltierelement zu drosseln, damit einer Überhitzung vorgebeugt ist.

Die Temperiervorrichtung 21 kann eine Steuereinheit 30 aufweisen. Die Steuereinheit 30 ist mit dem Peltierelement 22 sowie der Energiequelle verbunden, um das Peltierelement 22 und insbesondere dessen Heiz- bzw. Kühlleistung steuerbar regeln zu können. Die Steuereinheit 30 ist dazu ausgebildet, Temperaturen, mit welchen eine Körperregion gekühlt werden soll, zu überwachen und zu regeln. Dadurch sind Kühlabläufe beliebig gestaltbar, beispielsweise ist es möglich, eine Intervallkühlung oder eine Kühlung bei gleichbleibender Temperatur einzustellen.

Darüber hinaus erlaubt die Steuereinheit 30 eine Vorgabe bestimmter Maximalwerte und/oder Minimalwerte für die Temperatur, sodass ein gewünschter Temperaturbereich für eine individuelle Behandlung einstellbar ist. Es ist somit beispielsweise ein unerwünschtes Unterschreiten einer Minimaltemperatur vermeidbar. Weiter kann ein Patient eine beliebige Behandlungstemperatur individuell auf sein Befinden abstimmen.

Temperaturwerte und/oder Temperaturverläufe sind besonders bevorzugt variabel programmierbar bzw. einstellbar. Des Weiteren sind externe Signale vom Körper, Umwelt und/oder Umgebung in einen Programmverlauf miteinbeziehbar. Die Vorrichtung bzw. Steuereinheit 30 kann auch lernfähig ausgebildet sein. Dadurch sind Körperfunktionen wie beispielsweise ein Sauerstoffgehalt im Blut und/oder ein Hautwiderstand sowie externe Signale wie eine Lichtintensität, eine Lautstärke und/oder Vibrationen überwachbar. Ferner kann so auch ein Einfluss des Wetters überwacht werden. Aufgrund von Temperatur- und/oder Luftdruckänderungen variiert eine Wahrscheinlichkeit für beispielsweise Kopfschmerzen oder allgemein körperliches Wohlbefinden. Eine Ansteuerung der Steuereinheit 30 zur Regelung von speziellen Kühlverläufen erfolgt zweckmäßigerweise über ein Mobiltelefon oder dergleichen, welches z. B. über Bluetooth Low Energy mit der Steuereinheit verbunden ist. Die Steuereinheit 30 kann ein Bedienelement zur Temperatureinstellung sowie einen Gleichrichter zur Gleichstromerzeugung aufweisen. Sind mehrere Peltierelemente 22 vorgesehen, ist es zweckmäßig, wenn diese einzeln bzw. separat ansteuerbar sind, um unterschiedliche Kühlkurven an unterschiedlichen Körperbereichen zu ermöglichen. Insbesondere wird das zumindest eine Peltierelement 22 mit langsamen Pulsen von etwa 1 Hz, insbesondere etwa 0,7 Hz, besonders bevorzugt etwa 0,5 Hz oder weniger, angesteuert. Das Peltierelement 22 kann aber auch mit einer schnelleren Pulsfrequenz angesteuert werden. Ferner kann es vorgesehen sein, dass die Vorrichtung bzw. die Steuereinheit 30 mit einer App über ein Mobiltelefon, einen Tablet-PC oder dergleichen steuerbar ist. Eine solche App bietet verschiedene Kurven bzw. Daten für unterschiedliche Beschwerden an, aus welchen ein Patient bzw. Anwender wählen kann.

Wenn eine Temperatur genau geregelt werden soll, ist es weiter günstig, wenn zumindest ein Temperatursensor vorgesehen ist. Insbesondere ist es günstig, wenn mehrere, beispielsweise sechs, Temperatursensoren an unterschiedlichen Orten der Vorrichtung angeordnet sind. Die Temperatursensoren sind dabei bevorzugt nahe der Haut sowie an den Kühlmaterialien angeordnet. Insbesondere umfasst die zumindest teilweise flexible Leiterplatte einen oder mehrere Temperatursensoren. Ferner kann zumindest ein Temperatursensor zur Messung einer Umgebungstemperatur ausgebildet sein. Die Leiterplatte kann darüber hinaus weitere elektronische Bauteile umfassen, beispielsweise Sensoren zur Bestimmung einer Lichtintensität oder von Körperfunktionen. Des Weiteren kann es günstig sein, wenn Aktorikeinrichtungen vorgesehen sind, beispielsweise eine Vibrationseinrichtung zur mechanischen Stimulation einer Körperpartie, auf der die Temperiervorrichtung 21 appliziert ist. Außerdem kann die Vibrationseinrichtung zur Benachrichtigung der Nutzer über haptische Ausgabesignale genutzt werden, beispielsweise um den Nutzer über eine bestimmte Nutzungsdauer in Kenntnis zu setzen.

Es kann von Vorteil sein, wenn die Steuereinheit 30 eine akustische Ausgabeeinrichtung aufweist. Die akustische Ausgabeeinrichtung kann zur Ausgabe diverser Indikatoren wie Warnsignalen und/oder einen Kühlungsfortschritt für einen Patienten oder einen behandelnden Arzt ausgebildet sein.

In alternativen Ausführungsvarianten der Temperiervorrichtung 21 können zwei oder mehr Peltierelemente 22 vorgesehen sein, die jeweils in einem eigenen Aufnahmebereich 23 eingebracht sind. Zudem kann die Steuereinheit 30 zur individuellen Ansteuerung der jeweiligen Peltierelemente 22 vorgesehen sein. Die Steuereinheit 30 regelt eine Heizbeziehungsweise Kühlleistung der unterschiedlichen Peltierelemente 22 anhand einer von verschiedenen Temperatursensoren 31 erfassten Temperaturen der Peltierelementoberflächen in den jeweiligen Aufnahmebereichen. Zudem kann die Steuereinheit 30 bei Erreichen einer Zieltemperatur der Peltierelementoberfläche die Heizbeziehungsweise Kühlleistung der Peltierelemente 22 derart regeln, dass die Temperatur der Peltierelementoberfläche im Wesentlichen konstant gehalten wird. Beispielsweise ist es auch möglich, in einem "Boost"-Modus die Heizleistung der Peltierelemente 22 für eine gewisse Zeitspanne, zum Beispiel zu Beginn eines Heizvorgangs, deutlich zu erhöhen, um die Wartezeit bis zur Erreichung der Zieltemperatur an den Peltierelementoberflächen zu verkürzen.

Ferner ist die Steuereinheit 30 dazu ausgebildet, eine von einem Umgebungstemperatursensor gemessene Umgebungstemperatur bei der geregelten Heizbeziehungsweise Kühlleistung zu berücksichtigen. Die Steuereinheit 30 erhöht, verringert oder unterbindet die Heiz- beziehungsweise Kühlleistung in vorbestimmten Temperaturbereichen der Umgebungstemperatur. So kann exemplarisch in einer Umgebung, deren Temperatur höher ist als beispielsweise 55 °C, das weitere Erwärmen der Hautkontaktfläche 23 verhindert werden, da in diesem Fall von einer Fehlfunktion oder Falschbedienung auszugehen ist. Alternativ oder zusätzlich ist die Steuereinheit 30 ferner dazu ausgebildet, die Heiz- beziehungsweise Kühlleistung der zwei oder mehr Peltierelemente 22 phasenweise nur einem der Peltierelemente 22 zuzuführen. Die vorliegende Erfindung ist jedoch nicht auf die Ausführungsvariante mit einem Peltierelement 22 beschränkt, sondern kann auch nur einige der beschriebenen Merkmale aufweisen. Somit kann eine Temperiervorrichtung 21 beispielsweise mit zwei oder mehr Peltierelementen 22 vorgesehen sein, wobei beispielsweise diese auch unabhängig voneinander in Betrieb sind.

Die Steuereinheit 30 weist vorzugsweise zudem eine Kommunikationsschnittstelle auf, welche dazu ausgebildet ist, eine Datenverbindung mit einer Rechnereinheit herzustellen. Die Rechnereinheit kann beispielsweise als Smartphone, Server oder Tabletcomputer ausgebildet sein, wobei die Steuereinheit 30 dazu ausgebildet ist anhand von, von der Rechnereinheit über die Kommunikationsschnittstelle empfangenen Daten eine Leistung des Peltierelements 22 zu regulieren.

Die Beschichtung oder Folierung 32 der Temperiervorrichtung 21 erstreckt sich im Beispiel der Fig. 1 über den Aufnahmebereich der Gehäusebasis 35 hinaus und bedeckt zumindest bereichsweise auch einen Auflagerahmen 33. Eine Fixiereinrichtung 34 ist hier beispielhaft als ein oder mehrere Schraubverbindungen ausgebildet, wobei die Gehäusebasis 35 entsprechende Gewindebohrungen aufweist, um die Schraubverbindungen zu fixieren. Ferner ist die Fixiereinrichtung 34 so ausgebildet, dass der Freiraum unterhalb der Fixiereinrichtung 34 und oberhalb der Gehäusebasis 35 im Bereich des Aufnahmebereichs der Gehäusebasis 35 für das Peltierelement 22 eine geringere Höhe als das Peltierelement 22 selbst aufweist.

Dadurch kann bei einem Einbringen des Peltierelement 22 in diesen Freiraum Druck durch die Fixiereinrichtung 34 auf das Peltierelement 22 ausgeübt werden, die das Peltierelement 22 fest an einen darunter liegenden Kühlkörper presst und auf diese Weise den thermischen Kontakt mit der Gehäusebasis 35 bzw. dem Kühlkörper verbessert. Wie in Fig. 1 dargestellt ist, ragt die Fixiereinrichtung 34 folglich nicht über die Oberkante der Seitenwände im zurückgesetzten Bereich und damit nicht über die obere Isolationsplatte 25 des Peltierelements 22 hinaus.

Die Gehäusebasis 35 kann einen Auflagerahmen 33 aufweisen, welcher um den oberen Rand der Gehäusebasis 35 unter der Hautkontaktfläche 23 herum verläuft. Der Auflagerahmen 33 kann ein integraler Teil der Gehäusebasis 35 sein oder alternativ als separates Bauteil am Gehäuserahmen geeignet befestigt sein, beispielsweise durch einen Klippmechanismus, Schraubverbindungen oder Klebstoff.

Optional kann die Fixiereinrichtung 34 mehrere weitere Bauteile aufweisen. In Fig. 1 enthält die Fixiereinrichtung 34 exemplarisch eine Klemmbacke sowie ein mechanisches Übertragungselement. Das Übertragungselement ist in einer Normalenrichtung der Isolationsplatten 24, 25 des Peltierelements 22 betrachtet (in der Fig. 1 von unten nach oben verlaufend) zwischen den Isolationsplatten 24 und 25 formschlüssig positioniert. Dabei stützt sich das mechanische Übertragungselement an einer hervorstehenden inneren Kante der Isolationsplatten 24 und 25 ab. Die Klemmbacke ist an der Gehäusebasis 35 angebracht, wobei die Klemmbacke das mechanische Übertagungselement in Richtung der Basis drückt und somit die untere Isolationsplatte 24, an welcher sich das Übertragungselement abstützt, an die Gehäusebasis 35 presst.

Wie weiter oben erläutert, können vorzugsweise ferner ein oder mehrere Temperatursensoren 31 vorgesehen werden. Ein Temperatursensor 31 kann beispielsweise zwischen dem Peltierelement 22 und der Beschichtung 32 vorgesehen werden. Insbesondere kann ein Temperatursensor 31 am Rande des Aufnahmebereichs 23 angeordnet werden. Beispielsweise kann ein Temperatursensor 31 auch auf der Beschichtung 32 positioniert oder in einer Aussparung der Beschichtung 32 positioniert.

In einer besonderen Ausführungsvariante kann der Temperatursensor 31 in dem Peltierelement 22 eingebettet sein. Wie in Fig. 2 beispielhaft illustriert, kann das Peltierelement 22 beispielsweise durch zwei um etliche Millimeter voneinander beabstandete quadratische oder rechteckige Isolationsplatten aus keramischem Aluminiumoxid gebildet werden, zwischen denen die Halbleiterelemente in einem vorgegebenen Muster eingelötet sind. Hierzu kann das Muster der eingelöteteten Halbleiterelemente eine Aussparung am Rand des Peltierelements 22 bilden, welche sich um eine bestimmte Distanz zwischen den Isolationsplatten 24 und 25 in das Peltierelement 22 hinein erstreckt.

Fig. 2 zeigt eine schematische Darstellung von zwei Peltierelementen 22 mit jeweils einem integrierten Temperatursensor 31. Der Temperatursensor 31 kann beispielsweise bis etwa zur geometrischen Mitte des Peltierelements 22 hineinragen. Wahlweise ragt der Temperatursensor 31 bis etwa 1/3 oder 1/4 der Länge des Peltierelements 22 hinein. Zwei oder mehrere Temperatursensoren 31 können optional mittels eines Steges miteinander mechanisch und/oder elektronisch verbunden sein. Optional können mehrere Temperatursensoren 31 vorgesehen sein, welche wahlweise in den zuvor genannten Positionen oder alternativen Positionen vorgesehen sind.

Fig. 3 zeigt eine schematische Seitenansicht eines in der Konfiguration der Fig. 2 einsetzbaren Temperatursensors 31 in einem unverbauten Zustand. Der Temperatursensor 31 umfasst eine Leiterplatte 36 und einen negativen Temperaturkoeffizient (NTC)-Sensor 37 bzw. Heißleiter oder NTC-Widerstand 37. Der NTC-Sensor bzw. NTC-Widerstand 37 ist vorzugsweise in einem vorderen Bereich der Leiterplatte 36, das heißt in einem Bereich der Spitze der Leiterplatte 36 angeordnet. Auf diese Weise kann der NTC-Sensor bzw. NTC-Widerstand 37 eine Temperatur in einem Zentrum des Peltierelements 22 messen. Ferner ist der NTC-Sensor bzw. NTC-Widerstand 37 vorzugsweise auf einer unteren Seite der Leiterplatte 36 angebracht. Dennoch ist nicht ausgeschlossen, dass der NTC-Sensor bzw. NTC-Widerstand 37 an einer oberen oder seitlichen Seite der Leiterplatte 36 angeordnet ist.

Optional kann der Temperatursensor 31 mehrere NTC-Sensoren 37 aufweisen. In Fig. 3 sind beispielhaft zwei NTC-Sensoren 37 vorgesehen. Dabei können beide NTC-Sensoren 37 auf derselben Seite angeordnet sein, um beispielsweise eine stabilere Positionierung zu unterstützen. Statt zweier NTC-Sensoren 37 können auch ein NTC-Sensor 37 und ein entsprechend montierter Dummy-Sensor eingesetzt werden, wobei der Dummy-Sensor nicht zur Temperaturmessung eingesetzt wird.

Darüber hinaus umfasst der Temperatursensor 31 eine Spangenfedereinrichtung 38. Diese Spangenfedereinrichtung 38 ist auf einer von dem NTC-Sensor 37 abgewandten Seite der Leiterplatte 36 befestigt.

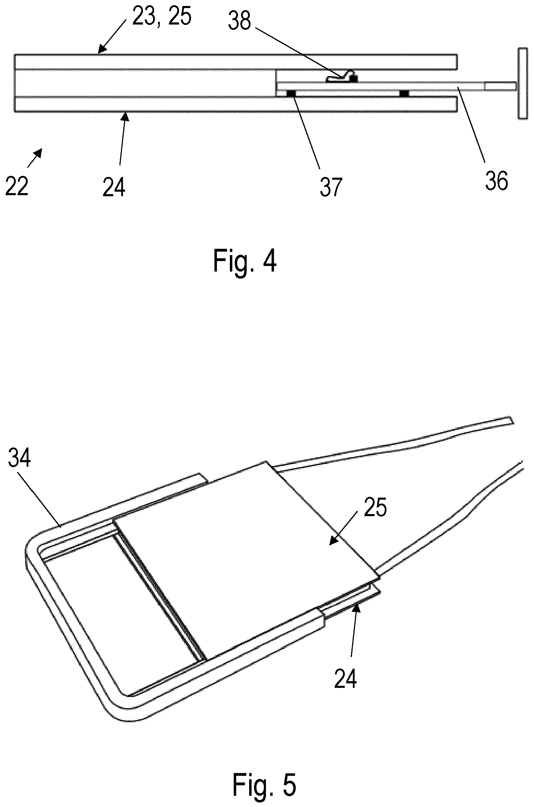

Fig. 4 zeigt eine im Schnitt dargestellte Seitenansicht eines Peltierelements 22 mit einem zwischen den Isolationsplatten 24 und 25 des Peltierelements 22 integrierten Temperatursensor 31.

Der Temperatursensor 31 weist im Wesentlichen die Merkmale des Temperatursensors 31 in einem unverbauten Zustand gemäß dem Ausführungsbeispiel nach Fig. 3 auf. Entsprechend des integrierten, also verbauten Zustandes ist die Spangenfedereinrichtung 38 des Temperatursensors 31 im Ausführungsbeispiel der Fig. 4 mit einer Druckspannung beaufschlagt. Somit drückt die Spangenfedereinrichtung 38 die Leiterplatte 36 nach unten und folglich die auf der von der Spangenfedereinrichtung 38 abgewandten Seite angeordneten NTC-Sensoren 37 auf die Isolationsplatte 24. Alternativ kann der Temperatursensor 31 um 180 ° gedreht in Bezug auf seine Längsachse in dem Peltierelement 22 integriert sein. Auf diese Weise ist die Temperatur der Isolationsplatte 25, die mit einem Aufnahmebereich der Gehäusebasis 35 korrespondiert, messbar.

Zur Integrierung des Temperatursensors 31 kann die Aussparung am Rand des Peltierelements 22, welche sich um eine bestimmte Distanz zwischen den Isolationsplatten 24 und 25 in das Peltierelement 22 hinein erstreckt, in ihrem Volumen an die Abmessungen des Temperatursensors 31 angepasst, so dass der Temperatursensor 31 zumindest teilweise, insbesondere vollständig, in die Aussparung hineinpasst. Optional kann das Peltierelement 22 zumindest abschnittsweise, insbesondere in dem Aufnahmebereich 23, eine Beschichtung 32 aufweisen.

Fig. 5 zeigt eine schematische Perspektivansicht eines Peltierelements 22 mit einer Fixiereinrichtung 34, welche das Peltierelement 22 zwischen den Isolationsplatten 24 und 25 formschlüssig zumindest abschnittsweise umschließt.

Die Fixiereinrichtung 34 ist beispielhaft als rechteckige Spange mit abgerundeten Ecken ausgebildet, jedoch nicht auf diese Form beschränkt. Sie kann ebenso U-förmig, V-förmig, hufeisenförmig oder dergleichen ausgebildet sein. Ferner kann die Fixiereinrichtung 34 oder ein Bauteil der Fixiereinrichtung 34 seitlich auf das Peltierelement 22 aufschiebbar, anlegbar, anklippbar oder vergleichbar angebracht sein. Wahlweise umschließt die Fixiereinrichtung 34 das Peltierelement 22 zumindest abschnittsweise, zum Beispiel an zwei, drei oder vier seitlichen Seiten. Dabei kann die Fixiereinrichtung 34 einteilig oder mehrteilig ausgebildet sein.

Peltierelemente wie hierin beispielhaft beschrieben sind thermoelektrische Module (TEM), welche thermoelektrische Halbleitermaterialien in Paaren unterschiedlichen Dotierungstyps (n-Typ und p-Typ) aufweisen. Diese thermoelektrischen Halbleitermaterialien werden zwischen zwei Keramikplatten eingebettet. Thermoelektrische Halbleitermaterialien, die im Allgemeinen in TEMs verwendet werden, können beispielsweise Legierungen aus BismutTellurid (Bi₂Te₃), Blei-Tellurid (PbTe), Silizium-Germanium (SiGe) und Bismut-Antimon (BiSb) aufweisen. Bi₂Te₃ wird aufgrund der ungleichen Elektronendichte häufig eingesetzt. Ein typisches TEM enthält eine strukturierte Anordnung von Halbleiterkugeln, Halbleitersäulen oder anders geformten Halbleiterelementen in Paaren unterschiedlichen Dotierungstyps (n-Typ und p-Typ), welche elektrisch in Reihe, aber thermisch parallel gekoppelt sind. Die strukturierte Anordnung erstreckt sich in einer flächigen Ausdehnung zwischen zwei mechanisch stabilen Isolationsplatten, meist aus keramischen Materialien wie beispielsweise Aluminiumnitrid (AlN) und Berylliumoxid (BeO) oder keramischem Al₂O₃.

Das TEM arbeitet nach den Prinzipien des Peltier-Effekts. Das bedeutet, dass beim Anlegen einer elektrischen Spannung an zwei Enden eine Halbleitermaterialelementes eine Temperaturanpassung erfolgt, die dazu führt, dass sich zwischen den Oberflächen der sich gegenüberliegenden, parallel zueinander verlaufenden Isolationsplatten bei einem Stromfluss durch die elektrisch in Reihe geschalteten Halbleiterelemente eine Temperaturdifferenz einstellt, so dass eine der Isolationsplatten gekühlt und die andere der Isolationsplatten erwärmt wird.

Um die Temperaturregelung des thermoelektrischen Moduls zu verbessern, ist in der strukturierten Anordnung von Paaren unterschiedlicher dotierter Halbleiterelemente eine nach außen offene Aussparung in der flächigen Ausdehnung eingebracht, d.h. zwischen den Isolationsplatten wird ein Hohlraum gebildet, dessen Decke und Boden die zwei Isolationsplatten bilden und dessen seitliche Begrenzungswände die umliegenden Halbleiterelemente bilden. In diesen Hohlraum kann ein Temperatursensor eingebracht werden, welcher ein Trägersubstrat, einen auf dem Trägersubstrat aufgebrachten Thermistor und elektrische Zuleitungen zu dem Thermistor aufweist. Das Trägersubstrat kann beispielsweise eine Leiterplatte (siehe Element 36 in Fig. 4) sein, auf der auf einer Seite ein Widerstand mit negativem Temperaturkoeffizienten (NTC-Widerstand) als Thermistor aufgebracht ist und auf der gegenüberliegenden Seite eine Spangenfedereinrichtung aufweist. Die Spangenfedereinrichtung dient dazu beim Einführen der Leiterplatte des Temperatursensors in die Aussparung von der Außenseite des thermoelektrischen Moduls eine Druckspannung auf den Temperatursensor auszuüben, so dass der Sitz des Temperatursensors innerhalb des Hohlraums mechanisch stabil ist.

## Patentansprüche

1. Temperiervorrichtung (21) zur dermalen Applikation, aufweisend:
eine Hautkontaktfläche (23);
zumindest ein mit der Hautkontaktfläche (23) verbundenes Peltierelement (22),
welches zwei parallele, voneinander beabstandete Isolationsplatten (24, 25) aufweist, zwischen denen dotierte Halbleiterelemente eingebracht sind, durch die elektrischer Strom geleitet werden kann; und
einen Temperatursensor (31), welcher in einer Aussparung des Peltierelements (22) zwischen den Isolationsplatten (24, 25) integriert und mit einer Temperaturmessschaltung außerhalb des Peltierelements (22) verbunden ist, und
welcher eine Leiterplatte (36) und einen auf einer Seite der Leiterplatte angebrachten negativen Temperaturkoeffizientsensor, NTC-Sensor, (37) aufweist,
**dadurch gekennzeichnet, dass**
der Temperatursensor (31) ferner eine Spangenfedereinrichtung (38) aufweist, welche auf einer von dem NTC-Sensor (37) abgewandten Seite der Leiterplatte (36) befestigt ist.

2. Temperiervorrichtung (21) nach Anspruch 1, wobei die Hautkontaktfläche (23) durch eine elektrisch nicht leitfähige Beschichtung oder Folierung, insbesondere einen Keramiklack oder eine Laminatfolie, der nach außen gekehrten Isolationsplatte (25) des Peltierelements (22) zum mechanischen Schutz der Peltierelementoberfläche gebildet wird.

3. Temperiervorrichtung (21) nach Anspruch 1 oder 2, ferner aufweisend:
eine Gehäusebasis (35), in der das Peltierelement (22) aufgenommen ist; und
eine Fixiereinrichtung (34), welche das Peltierelement (22) an der Gehäusebasis (35) fixiert.

4. Temperiervorrichtung (21) nach Anspruch 3, wobei die Fixiereinrichtung (34) das Peltierelement (22) zwischen den Isolationsplatten (24, 25) formschlüssig zumindest abschnittsweise umschließt.

5. Temperiervorrichtung (21) nach einem der Ansprüche 1 bis 4, ferner aufweisend eine Steuereinheit (30), welche mit dem Temperatursensor (31) und dem Peltierelement (22) verbunden ist, wobei die Steuereinheit (30) dazu ausgebildet ist, eine Heizbeziehungsweise Kühlleistung des Peltierelements (22) anhand einer von dem Temperatursensor (31) erfassten Temperatur des Peltierelements (22) zu regeln.

## Claims

1. Tempering device (21) for dermal application, having:
a skin contact surface (23);
at least one Peltier element (22) which is connected to the skin contact surface (23) and has two parallel insulation plates (24, 25) spaced apart from one another, between which doped semi-conductor elements are inserted, through which electric current can be conducted; and
a temperature sensor (31), which is integrated in a recess of the Peltier element (22) between the insulation plates (24, 25) and is connected to a temperature measuring circuit outside of the Peltier element (22), and which has a printed circuit board (36) and a negative temperature coefficient sensor, NTC sensor (37), fitted to one side of the printed circuit board, **characterised in that**
the temperature sensor (31) further has a clasp spring device (38), which is attached to one side of the printed circuit board (36) facing away from the NTC sensor (37).

2. Tempering device (21) according to claim 1, wherein the skin contact surface (23) is formed by an electrically nonconductive coating or film, in particular a ceramic coating or a laminate film, of the outwardly facing insulation plate (25) of the Peltier element (22) for mechanically protecting the Peltier element surface.

3. Tempering device (21) according to claim 1 or 2, further having:
a housing base (35), in which the Peltier element (22) is received; and
a fixing device (34), which fixes the Peltier element (22) to the housing base (35).

4. Tempering device (21) according to claim 3, wherein the fixing device (34) surrounds the Peltier element (22) between the insulation plates (24, 25) in a form-fitting manner at least in sections.

5. Tempering device (21) according to one of claims 1 to 4, further having a control unit (30), which is connected to the temperature sensor (31) and the Peltier element (22), wherein the control unit (30) is designed to regulate a heating or cooling capacity of the Peltier element (22) based on a temperature of the Peltier element (22) detected by the temperature sensor (31).

## Revendications

1. Dispositif de régulation de température (21) pour une application dermique, comportant :
une surface de contact avec la peau (23) ;
au moins un élément à effet Peltier (22) relié à la surface de contact avec la peau (23) et comportant deux plaques d'isolation parallèles (24, 25) espacées l'une de l'autre, entre lesquelles sont insérés des éléments semi-conducteurs dopés à travers lesquels du courant électrique peut être conduit ; et
un capteur de température (31) qui est intégré dans un évidement de l'élément à effet Peltier (22) entre les plaques d'isolation (24, 25) et qui est relié à un circuit de mesure de température à l'extérieur de l'élément à effet Peltier (22), et qui comporte une carte imprimée (36) et un capteur à coefficient de température négatif, capteur NTC, (37) monté sur une face de la carte imprimée,
**caractérisé en ce que**
le capteur de température (31) comporte en outre un dispositif à ressort (38) qui est fixé sur une face de la carte imprimée (36) opposée au capteur NTC (37).

2. Dispositif de régulation de température (21) selon la revendication 1, dans lequel la surface de contact avec la peau (23) est formée par un revêtement ou une feuille électriquement non conducteur/conductrice, en particulier un émail céramique ou une feuille stratifiée, de la plaque d'isolation (25) retournée vers l'extérieur de l'élément à effet Peltier (22) pour assurer la protection mécanique de la surface de l'élément à effet Peltier.

3. Dispositif de régulation de température (21) selon la revendication 1 ou 2, comportant en outre :
une base de boîtier (35) dans laquelle l'élément à effet Peltier (22) est reçu ; et
un dispositif de fixation (34) qui fixe l'élément à effet Peltier (22) sur la base de boîtier (35).

4. Dispositif de régulation de température (21) selon la revendication 3, dans lequel le dispositif de fixation (34) entoure par complémentarité de formes, au moins par sections, l'élément à effet Peltier (22) entre les plaques d'isolation (24, 25).

5. Dispositif de régulation de température (21) selon l'une des revendications 1 à 4, comportant en outre une unité de commande (30) qui est reliée au capteur de température (31) et à l'élément à effet Peltier (22), dans lequel l'unité de commande (30) est configurée pour réguler une puissance de chauffage ou de refroidissement de l'élément à effet Peltier (22) sur la base d'une température de l'élément à effet Peltier (22) détectée par le capteur de température (31).
